# EUROPEAN PATENT SPECIFICATION

(11) **EP 2 081 628 B1**
(45) Date of publication and mention of the grant of the patent: **12.06.2013**
(21) Application number: 07874399.4
(22) Date of filing: 16.10.2007
(51) Int. Cl.: A61M 25/04, A61M 27/00

(54) **NONEXPANDABLE STENT**
NICHT EXPANDIERBARER STENT
ENDOPROTHÈSE VASCULAIRE NON EXTENSIBLE

(30) Priority: 16.10.2006 US 852034 P
(43) Date of publication of application: 29.07.2009
(73) Proprietor: Cook Medical Technologies LLC, Bloomington, IN 47404 (US)
(72) Inventor: DUCHARME, Richard, W., Winston-salem, NC 27106 (US)
(74) Representative: Garratt, Peter Douglas
(86) International application number: PCT/US2007/081460
(87) International publication number: WO 2008/115271

(56) References cited:
- WO-A-00/66032
- WO-A-01/91668
- WO-A-96/11721
- WO-A-03/075795
- GB-A- 2 018 600
- US-A- 4 874 360
- US-A- 4 931 037
- US-A- 6 139 536
- US-A1- 2001 047 164
- US-A1- 2004 193 092

## Description

### TECHNICAL FIELD

The present disclosure is related generally to medical devices and more particularly to stents.

### BACKGROUND

Biliary and pancreatic cancers often are diagnosed when the patient presents specific symptoms characteristic of a blockage of either the patient's bile and/or pancreatic duct, such as jaundice. Typically, by the time symptoms appear in the patient, a tumor in the bile or pancreatic duct is at an advanced stage and is therefore inoperable. As a result, management of the cancer usually focuses on palliation of the symptoms. As an alternative to surgical bypass procedures for palliation, a stent or endoprosthesis may be positioned through the obstructed area so as to maintain a pathway for fluid flow across the obstruction.

Typically, stents used for drainage in the biliary tract are nonexpandable tubular structures formed from biocompatible polymers. When inserted, one end of the stent may be disposed distal of the ductal obstruction, and the other end may protrude into the duodenum. To anchor a biliary or pancreatic stent in place, one or both ends may have a curved "pigtail" configuration or may include flaps.

Reference is herein directed to WO 01/91668 which discloses an uretal stent having a pigtail at one end and at the other end a flared portion maintained in shape by an elastic ring or loop which may be formed of a shape-memory material.

For delivery into the duct, the stent may be advanced over a wire guide which has been positioned in the duct distal of the occluded site. The stent may pass through an endoscope disposed in the duodenum and then into the duct. Passage of the stent over the wire guide tends to temporarily straighten any curves in the stent (e.g., pigtails) for delivery into the duct. Once the wire guide is withdrawn, the stent may assume its curved configuration.

The curved configuration of a biliary or pancreatic stent is typically achieved by heat forming the stent after extrusion at elevated temperatures. Consequently, to facilitate fabrication and forming, it is generally desirable that the stent be made of a thermoplastic material that softens or flows when heated. Polymers that are not thermoplastics may not be amenable to processing by extrusion and heat forming.

On the other hand, some polymers that have desirable properties (e.g., biocompatibility, low durometer) are not thermoplastic. It would be desirable to be able to use such polymers to form biliary or pancreatic stents.

### BRIEF SUMMARY

A nonexpandable stent as claimed in claim 1, and a method of making the stent, as claimed in claim 11, are disclosed herein. The stents of the present disclosure may be formed from a wide range of polymers that have desirable properties, such as, for example, Thoralon.

the stent, as claimed in claim 1, includes a tubular body having a distal portion, a proximal portion, and a central longitudinal portion between the distal and proximal portions. The tubular body has a substantially nonexpandable diameter and comprises at least one securing element. The securing element includes a reinforcement member comprising a shape memory material. The securing element comprises a first configuration of the reinforcement member for delivery to a treatment site within a body vessel and a second configuration of the reinforcement member for deployment at the treatment site.

Also described is a method not being part of the present invention, of deploying a nonexpandable stent. To carry out the method, a stent comprising a tubular body having a distal portion, a proximal portion, a central longitudinal portion between the distal and proximal portions, and a substantially nonexpandable diameter, is provided. The tubular body comprises at least one securing element. The securing element includes a reinforcement member comprising a shape memory material. The stent is delivered to a treatment site in a body vessel. The securing element comprises a first configuration of the reinforcement member when the stent is being delivered. The stent is then deployed at the treatment site. The securing element comprises a second configuration of the reinforcement member when the stent is deployed.

In another aspect, the present invention consists in a method as claimed in claim 11, of making the nonexpandable stent referred to above, providing at least one reinforcement member comprising a nickel-titanium alloy shape memory material, heating the reinforcement member to impart a memory of a desired final shape to the reinforcement member; after the heating treating, holding the reinforcement member adjacent to a mandrel with a spacing therebetween along a length of the reinforcement member; applying a coating solution to the reinforcement member and the mandrel, curing the coating solution, thereby obtaining a polymer layer on the reinforcement member and the mandrel; removing the mandrel, thereby forming a nonexpandable stent including the reinforcement member within in a wall thereof, wherein the applying and curing are repeated sequentially to form successive polymer layers on the reinforcement member and the mandrel before removing the mandrel.

### BRIEF DESCRIPTION OF THE DRAWINGS

FIG. 1 is a schematic of a stent according to one embodiment in the pancreatic duct at a stricture;

FIG. 2A is a schematic of a securing element of the stent of FIG. 1 in a deployed configuration, according to one embodiment;

FIG. 2B is a cross-sectional schematic of section 2B-2B shown in FIG. 2A.

FIG. 3 is a schematic of a securing element of a stent in a deployed configuration, according to another embodiment;

FIG. 4 is a schematic of a securing element of a stent in a deployed configuration, according to another embodiment;

FIG. 5A is a schematic of a stent including two securing elements, according to one example

FIG. 5B is a cross-sectional schematic of a distal portion of the securing element of the stent shown in FIG. 5A;

FIG. 6 is a cross-sectional schematic of a stent including two securing elements;

FIGS. 7A-7B are cross-sectional views of a portion of a stent including a wire, according to one embodiment;

FIG. 8 is a cross-sectional view of a portion of a stent including a wire, according to another embodiment;

Figure 9 is a diagram of stress versus strain for an exemplary shape memory material at a temperature above an austenitic final temperature of the alloy;

Figure 10 is a transformation temperature curve for an exemplary shape memory material;

Figure 11 is a diagram of strain versus temperature for an exemplary shape memory material;

Figures 12A-12D show a method of deploying a stent according to one aspect; and

Figures 13A-13E show a method of deploying a stent according to another aspect.

Figures 14A-14D show a method of delivering and deploying a stent according to one aspect.

### DETAILED DESCRIPTION

### Definitions

As used in the following specification and the appended claims, the following terms will have the meanings ascribed below:

Martensite start temperature (Mₛ) is the temperature at which a phase transformation to martensite begins upon cooling for a shape memory material exhibiting a martensitic phase transformation.

Martensite finish temperature (M_{f}) is the temperature at which the phase transformation to martensite concludes upon cooling.

Austenite start temperature (Aₛ) is the temperature at which a phase transformation to austenite begins upon heating for a shape memory material exhibiting an austenitic phase transformation.

Austenite finish temperature (A_{f}) is the temperature at which the phase transformation to austenite concludes upon heating.

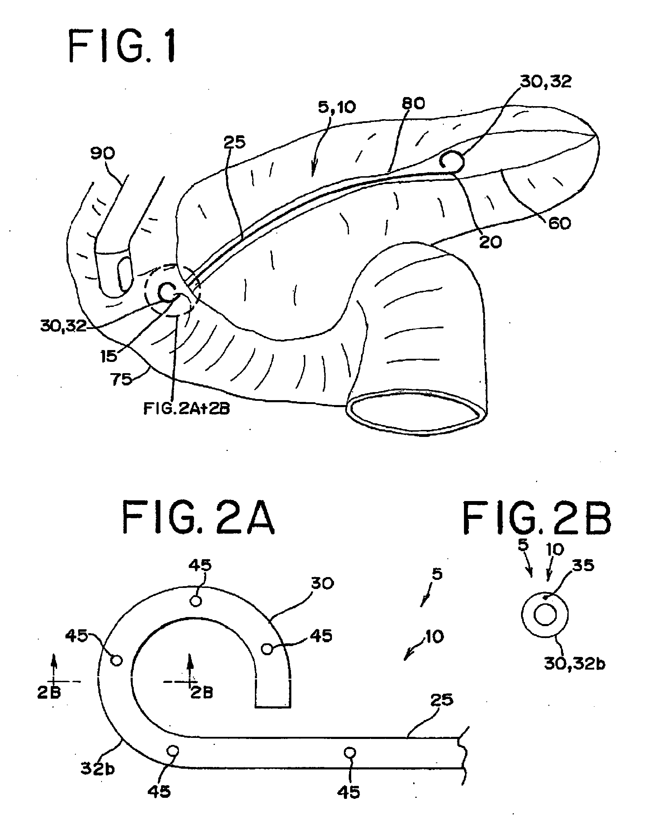

Figure 1 is a schematic of the nonexpandable stent 5 of the present disclosure according to one embodiment. The stent 5 is shown deployed in the pancreatic duct 60 crossing a treatment site or stricture 80. An endoscope 90 through which the stent 5 is directed en route to the stricture 80 is shown positioned in the duodenum 75.

The stent 5 includes a tubular body 10 having a substantially nonexpandable diameter. The tubular body 10 includes a proximal portion 15, a distal portion 20, and a central longitudinal portion 25 between the proximal portion 15 and the distal portion 20. The tubular body 10 includes at least one securing element 30. The securing element 30 includes at least one reinforcement member 35 made of a shape memory material. Preferably, the reinforcement member 35 is a wire. Alternatively, the reinforcement member 35 may be a tubular structure. The securing element 30 and the reinforcement member 35 are shown according to one embodiment in Figures 2A and 2B.

As shown in Figure 1, the securing element 30 has a deployment configuration of the reinforcement member 35 for deployment of the stent 5 at a treatment site in a body vessel or passageway. In the deployment configuration, the securing element 30 is configured to inhibit movement of the stent 5 with respect to the body vessel. The securing element 30 may anchor the stent 5 in position in the biliary or pancreatic duct, for example. Preferably, the securing element 30 extends in a direction away from the central longitudinal portion 25 of the tubular body 10 in the deployment configuration. the shape memory material of the reinforcement member 35 is a nickel-titanium alloy, and the reinforcement member 35 comprises an austenitic phase of the nickel-titanium alloy in the deployment configuration, as will be further discussed below.

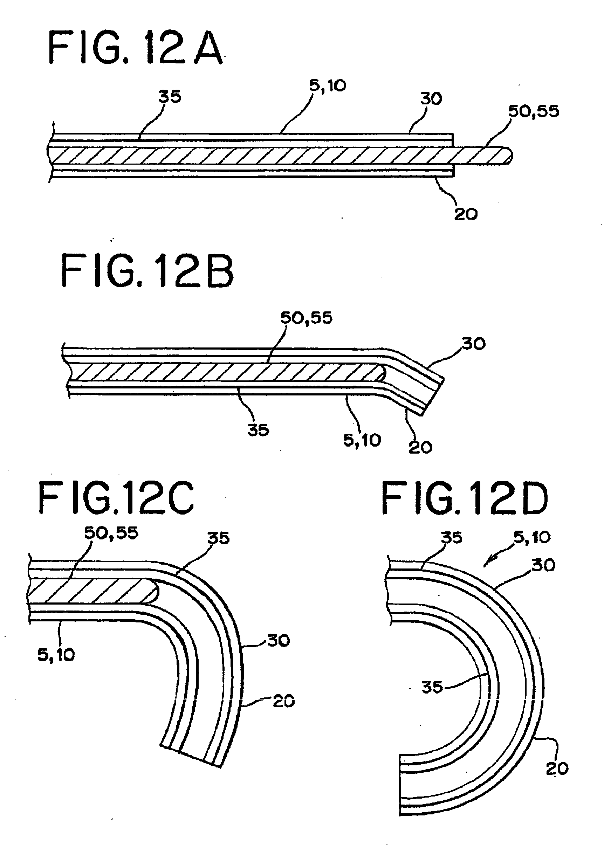

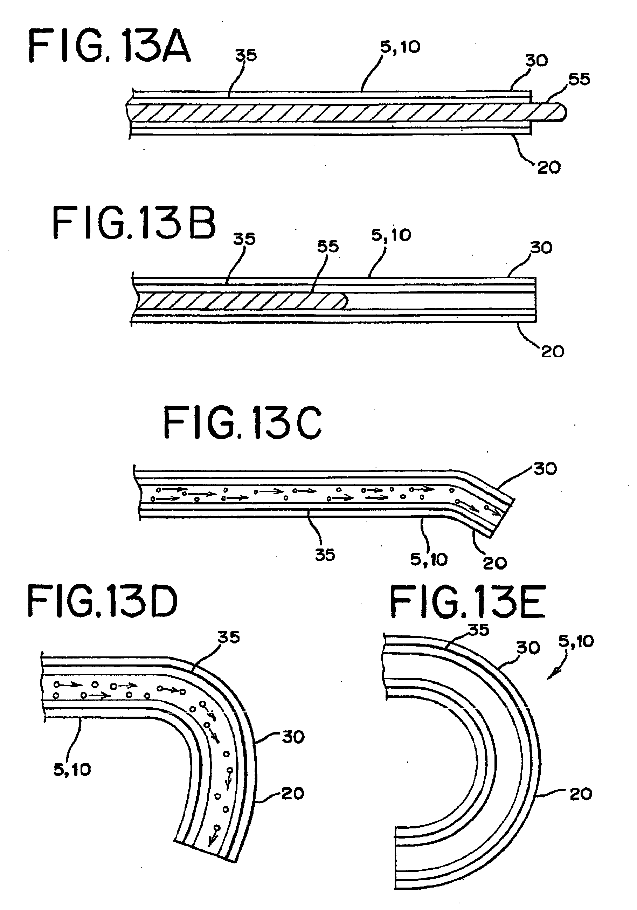

For delivery of the stent 5 into a body passageway, the securing element 30 has a delivery configuration of the reinforcement member 35, as shown in Figures 12A and 13A, for example. In the delivery configuration, the securing element 30 is configured to facilitate movement of the stent 5 through the body vessel. Preferably, the securing element 30 extends in a longitudinal direction of the tubular body 10 in the delivery configuration. the shape memory material of the reinforcement member 35 is a nickel-titanium alloy, and the reinforcement member 35 comprises a martensitic phase of the nickel-titanium alloy in the delivery configuration, as will be further discussed below.

The securing element 30 is disposed in at least one of the distal portion 20, the proximal portion 15, and the central longitudinal portion 25 of the tubular body 10. Preferably, the securing element 30 is disposed in at least one of the distal portion 20 and the proximal portion 15 of the tubular body 10.

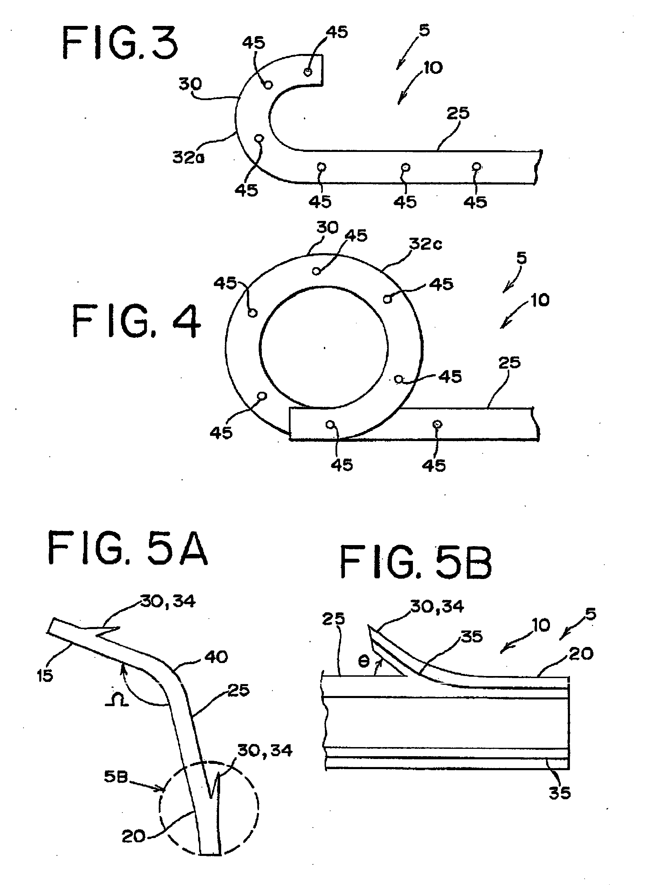

The securing element 30 may include a curve or "pigtail" 32 when the reinforcement member 35 is in the deployment configuration, according to one embodiment. For example, the securing element may include a curve or pigtail 32 of between about 180 degrees and about 270 degrees when deployed. A curve or pigtail 32a of about 270 degrees is shown for example in FIG. 2A. A curve or pigtail 32b of about 180 degrees is shown for example in FIG. 3. Alternatively, the curve or pigtail 32 may be between about 270 degrees and about 360 degrees when deployed. A curve or pigtail 32c of about 360 degrees is shown for example in FIG. 4. In another example, the securing element may include a curve or pigtail of greater than 360 degrees.

According to another example not being part of the invention, the securing element 30 may be one or more flaps 34, as shown in FIGS. 5A and 5B. The configuration of the flaps 34 is determined by the orientation of the reinforcement member 35. When deployed, the flaps 34 preferably flare away from the central longitudinal portion 25 at an included angle θ in the range of from about 2 to about 60 degrees. More preferably, the flaps 34 flare away from the central longitudinal portion 25 at an included angle θ in the range of from about 5 to about 45 degrees when deployed. The flaps 34 are typically from about 0.5 mm to about 5 mm in length.

According to another embodiment, the securing element 30 may be a bend 40 in the central longitudinal portion 25 when the reinforcement member 35 has the deployment configuration, as shown for example in FIG. 5. The bend 40 may have an included angle Q in the range of from about 95° to about 175°. Preferably, the bend 40 has an included angle Ω in the range of from about 105° to about 165°.

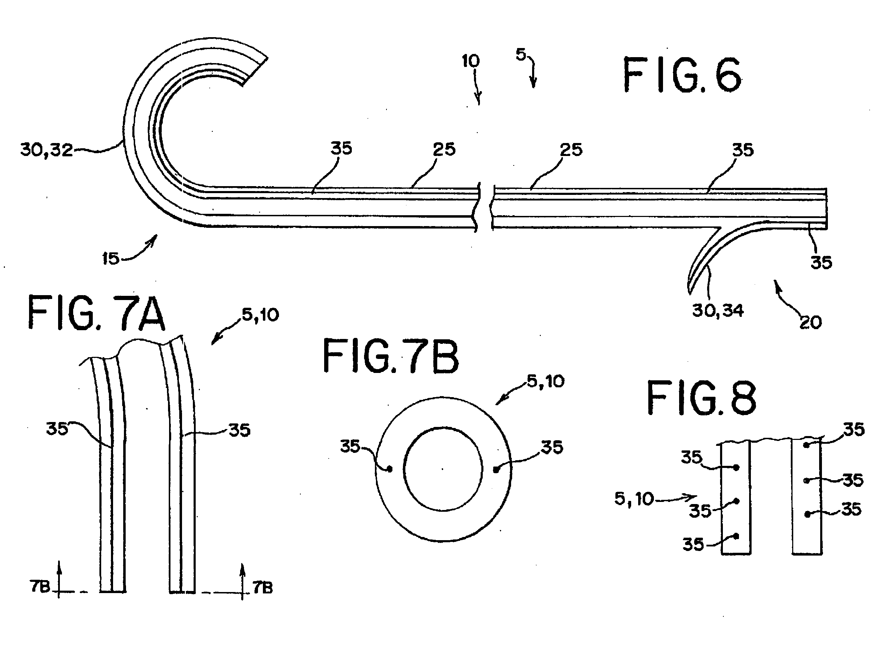

Alternatively, the securing element 30 may include a combination not being part of the present invention, of elements, such as flaps 34 and pigtails 32. For example, referring to Figure 6, the securing element 30 at one of the distal portion 20 and the proximal portion 15 may be a pigtail 32, and the securing element at the other portion may be one or more flaps 34. Other securing elements 30 may also be used for the stent 5 of the present disclosure. The securing element 30 may have any shape suitable for anchoring the stent 5 in position at the desired site within the duct, such as a hook-like or a corkscrew-like configuration, for example. The tubular body 10 of the stent 5 may include two, three, four, five, six or more securing elements 30.

the reinforcement member 35 extends along at least a portion of a length of the securing element 30. According to one embodiment, the reinforcement member 35 may extend along the entire length of the securing element 30, as shown for example in Figure 6. the reinforcement member 35 is disposed such that the configuration of the securing element 30 is altered by a change in the configuration of the reinforcement member 35. Consequently, when the reinforcement member 35 has a specified configuration, the securing element 30 has the same configuration. The reinforcement member 35 extends along at least a portion of a length of the tubular body 10. For example, the reinforcement member 35 may extend from the distal portion 20 to the proximal portion 15 of the tubular body 10. Alternatively, the reinforcement member 35 may extend along the length of the securing element 30 to one of the distal portion 20 and the proximal portion 15 of the tubular body 10. According to an alternative embodiment, the reinforcement member 35 may extend only along the length of the securing element 30.

The stent 5 may include one or more reinforcement members 35. For example, the stent 5 may include two, three, four, or five reinforcement members 35, as indicated in Figures 7A and 7B. When viewed in cross-section, the reinforcement members 35 may be positioned symmetrically about the circumference of the tubular body 10. Alternatively, the reinforcement members 35 may be nonsymmetrically arranged about the circumference of the tubular body 10. According to one embodiment, the reinforcement member(s) 35 may extend in a longitudinal direction of the tubular body 10 when the stent 5 is undeployed. The reinforcement member 35 may also extend in a circumferential direction of the tubular body 10. For example, the reinforcement member 35 may be disposed in a helical configuration, as shown in FIG. 8. In another example, the reinforcement member(s) 35 may have a braided configuration.

Preferably, the reinforcement member 35 is a wire. For example, the reinforcement member 35 may be a round wire with a circular cross-section. Alternatively, the reinforcement member 35 may be a flat wire with a rectangular cross-section. Other curved or polygonal cross-sections are also possible. The reinforcement member 35 may alternatively be a tubular structure. The diameter or width of the reinforcement member 35 (in the case of a tubular structure, the outer diameter specifically) is preferably less than the wall thickness of the tubular body 10. According to one embodiment, the diameter or width of the reinforcement member 35 is approximately half the wall thickness of the tubular body 10. For example, the diameter or width of the reinforcement member 35 may be in the range of from about 0.1 to about 0.5 mm, although other values are possible. The stent 5 may be, for example, a 10 French stent with an outer diameter of about 3.4 mm, an inner diameter of about 2.5 mm, and a wall thickness of about (3.4 mm-2.5 mm)/2 ∼ 0.45 mm. In this example, it may be advantageous for the reinforcement member 35 to have a diameter of about 0.23 mm. Alternatively, the stent 5 may be a 3 French stent with an outer diameter of about 1 mm, an inner diameter of about 0.056 mm, and a wall thickness of about (1 mm-0.056 mm)/2 ∼ 0.47 mm. In this case, the reinforcement member 35 may advantageously have a diameter of about 0.24 mm.

The stent 5 comprises polymer. The tubular body 10 of the stent 5 may be made of one or more polymers. The polymer may be a thermoplastic or thermosetting polymer. According to one example, the polymer is a biocompatible polyurethane, such as Thoralon. Thoralon is available from Thoratec Corp. (Pleasanton, CA) and is described in U.S. Pat. Nos. 4,675,361 and 6,939,377. Thoralon is a polyurethane base polymer blended (referred to as BPS-215) with a siloxane containing surface modifying additive (referred to as SMA-300). The concentration of the surface modifying additive may be in the range of 0.5% to 5% by weight of the base polymer. The SMA-300 component is a polyurethane comprising polydimethylsiloxane as a soft segment and the reaction product of diphenylmethane diisocyanate (MDI) and 1,4-butanediol as a hard segment. A variety of other biocompatible polyurethanes may also be used as the polymer. These include polyurethane ureas that preferably include a soft segment and include a hard segment formed from a diisocyanate and diamine. For example, polyurethane ureas with soft segments such as polytetramethylene oxide, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used.

the reinforcement member 35 is embedded in the polymer. The tubular body 10 of the stent 5 may include drainage holes 45 along its length to facilitate the flow of body fluids into the lumen of the stent 5 for drainage out of the duct. Exemplary drainage holes 45 are shown in Figures 2A, 3 and 4.

Typically, the stent, a wire guide, and a pushing catheter are elements of a stent introduction system for delivering the stent within a body vessel or duct. A guiding catheter may also be used. The stent may have an outer diameter in the range of from about 3 French (1 mm) to about 12 French (4 mm), and an inner diameter sized to receive a wire guide and, in some embodiments, the guiding catheter. Generally, guiding catheters may be used with larger diameter stents. The guiding catheter may have an outer diameter or French size that can be accommodated within the inner diameter of the stent. The stent may include a distal region having a reduced inner diameter that serves as a distal stop when the guiding catheter is inserted into the stent. The guiding catheter may have an outer diameter that is similar to the outer diameter of the pushing catheter. If a guiding catheter is not used, the outer diameter of the stent may be similar to the outer diameter of the pushing catheter. The wire guide may be about 0.09 m (0,035 inch) in diameter, or another suitable size.

The stent may have a length suitable for placement and securing in the duct of interest. According to one embodiment, the length of the stent, as measured between the securing elements or between one end and a securing element, may be longer than the distance from the duodenum to the treatment site or stricture within the duct. Typically, the length of the stent is about 1 cm longer than the distance from the duodenum to the proximal margin of the stricture. For example, if the stricture lies within the pancreatic duct about 6.0 cm away from the duodenum, a stent of about 6.5 cm or 7.0 cm in length may be appropriate. According to another embodiment, the length may be longer than the distance from the duodenum to the terminus or tail of the duct. For example, in the case of a pancreatic duct measuring about 16.0 cm in length, a stent of about 16.5 cm or 17.0 cm in length may be appropriate.

The shape memory material of the reinforcement member 35 undergoes a reversible phase transformation that allows a previous shape to be "remembered" and recovered from another shape. A securing element 30 including the reinforcement member 35 changes from one configuration (*e.g.*, delivery configuration) to another (*e.g.*, deployment configuration) when the shape memory material of the reinforcement member 35 undergoes the phase transformation. For example, the shape memory material may undergo a transformation between a lower temperature martensitic phase and a high temperature austenitic phase. According to one embodiment, the shape memory material is a nickel-titanium alloy.

The delivery configuration of the reinforcement member 35 comprises the martensitic phase of the shape memory material. The deployment configuration of the reinforcement member 35 comprises the austenitic phase of the shape memory material. Austenite is characteristically the stronger phase, and martensite may be deformed up to a recoverable strain of about 8%. Strain introduced in the reinforcement member 35 in the martensitic phase to achieve the delivery configuration of the securing element 30 may be recovered upon completion of a reverse phase transformation to austenite, allowing the reinforcement member 35, and thus the securing element 30, to return to a previously-defined shape (the deployment configuration). The forward and reverse phase transformations may be driven by the application and removal of stress (superelastic effect) and/or by a change in temperature (shape memory effect). According to an alternative example, the delivery configuration of the reinforcement member 35 may comprise the austenitic phase of the shape memory material, and the deployment configuration of the reinforcement member 35 may comprise the martensitic phase.

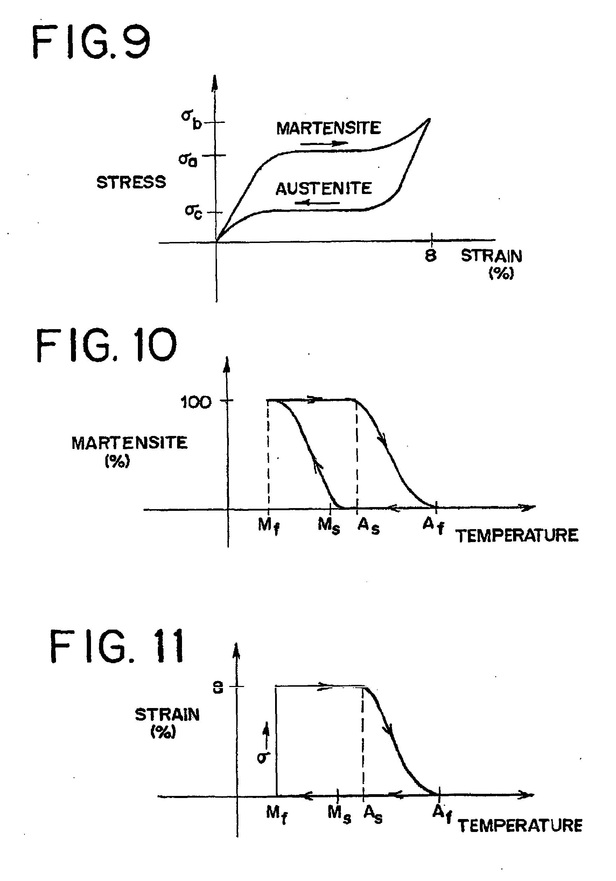

The stress-strain diagram in Figure 9 illustrates the superelastic effect for an exemplary nickel-titanium alloy at a temperature above the austenitic final temperature (A_{f}) of the alloy. Upon application of a stress σₐ, an alloy in a first configuration begins to transform from austenite to martensite. The martensitic phase of the alloy can accommodate several percent strain at a nearly constant stress. At a stress of σ_{b}, which corresponds to 8% strain in this example, the martensitic transformation is complete and the alloy has been deformed to a second configuration. Upon release of the stress, the martensite begins to transform back to austenite and the alloy recovers the strain at a lower plateau stress of σ_{c}. The nickel-titanium alloy thus returns to the first configuration.

Figure 10 shows a typical transformation temperature curve for an exemplary nickel-titanium shape memory alloy, where the y-axis represents the amount of martensite in the alloy and the x-axis represents temperature. At or above a temperature of A_{f}, the nickel-titanium alloy has a fully austenitic structure. Following the arrows, the alloy may be cooled to a temperature of Mₛ, at which point the transformation to the martensitic phase begins. Further cooling leads to an increase in the percentage of martensite in the material, ultimately leading to a fully martensitic structure at a temperature of M_{f}, as shown in Figure 10.

Now referring also to Figure 11, which shows strain versus temperature for an exemplary nickel-titanium shape memory alloy, the fully martensitic structure attained at a temperature of M_{f} may be strained from a first configuration to a second configuration (as shown by the stress symbol σ). The alloy may accommodate several percent recoverable strain (8% in this example). To reverse the phase transformation and recover the strain, the temperature of the alloy must be increased. Again following the arrows, the nickel-titanium alloy may be warmed to a temperature of Aₛ, at which point the alloy begins to transform to the austenitic phase. Upon further heating, the transformation to austenite progresses and the alloy gradually recovers the first configuration. Ultimately, at a temperature of A_{f} or higher, the material has completed the return transformation to the austenitic phase (0% martensite) and has fully recovered the 8% strain.

Generally, the shape memory memory effect is one-way, which means that the spontaneous change from one configuration to another occurs only upon heating. As illustrated in Figure 11, to obtain a second configuration at a temperature below a transition temperature, it is generally necessary to apply stress. However, it is possible to obtain a two-way shape memory effect, in which a shape memory material spontaneously changes shape upon cooling as well as upon heating. According to one aspect, the shape memory material of the reinforcement member 35 may exhibit two-way shape memory behavior. For example, the delivery configuration of the securing element 30 may be attained by cooling to a temperature at or below M_{f} without application of an external stress.

Referring to Figures 12A to 12D, the superelastic effect may be used to deploy the stent 5. In other words, the shape memory material transforms from the martensitic phase to the austenitic phase for deployment of the stent 5 in response to removal of an applied stress. According to this aspect, the stent 5 may be maintained in the delivery configuration by a constraining member 50, such as a stiff wire guide 55 underlying the stent 5, as shown in the figures, or a sheath overlying the stent. An underlying wire guide 55 may be sufficient if the securing element(s) 30 are pigtails 32 or similar structures that are integral with the tubular body 10, whereas an overlying sheath may be required if the securing element(s) are flaps 34. The shape memory material comprises the martensitic phase when constrained by the constraining member 50. The reinforcement member 35 and consequently the securing element 30 of the stent 5 may change from the delivery configuration to the deployment configuration (e.g., pigtails) at the treatment site when the constraining member 50 is removed or retracted and the martensite transforms to austenite, as illustrated in Figures 12A to 12D. that the shape memory material of the reinforcement member 35 has an austenitic final temperature (A_{f}) which is less than or equal to body temperature (37°C) so that removal of the constraining member 50 (wire guide 55) is sufficient to trigger the transformation to the austenitic phase when the stent 5 is positioned at the treatment site. For example, the shape memory material may have a value of A_{f} in the range of from about 27°C to 37°C. Alternatively, A_{f} may range from about 32°C to 37°C. It is also possible for A_{f} to be less than 27°C. In addition, if Aₛ is above ambient temperature (e.g., about 20°C), the shape memory material of the stent 5 may be martensitic at room temperature.

Referring to Figures 13A to 13E, the shape memory effect may be utilized to deploy the stent 5. According to this aspect, a constraining member 50 may not be used. A shape memory material having a value of A_{f} which is greater than body temperature (37°C) but below a temperature that may be damaging to tissue may be chosen for the reinforcement member 35. For example, the shape memory material may have a value of A_{f} in the range of from about 38°C to about 5$°C. Or, A_{f} may range from about 38°C to about 50°C. Accordingly, the stent 5 has a martensitic structure as it is advanced through the body. When the stent 5 is in place at the treatment site, the stent 5 (reinforcement member 35) is warmed up to a temperature of A_{f} or higher. Consequently, the martensite transforms austenite and the securing element(s) 30 reaches the deployment configuration to anchor the stent 5 into the duct. The warming may entail, for example, removing the wire guide 55 and flushing a warm biocompatible fluid (e.g., warm saline) through the lumen of the stent, as illustrated in Figures 13A to 13E. Alternatively, the wire guide 55 or optional guiding catheter may include a lumen to accommodate the flow of fluid. According to this aspect, the wire guide 55 may be used as a guide for positioning the stent 5 but is not needed as a constraining member 50 to maintain the delivery configuration. Once the deployment configuration has been obtained, the heating may be halted and the stent 5 may remain in the duct in the deployment configuration. To maintain the austenitic structure of the shape memory alloy while the stent 5 is in place within the duct, the shape memory alloy may be chosen such that M_{f}, and preferably Mₛ, are below body temperature. Because austenite is stronger and less easily deformed than martensite, the austenitic phase of the shape memory alloy is retained when the stent 5 is deployed in the second configuration. If M_{f} and Mₛ are not below body temperature, it may be necessary to continuously heat the stent 5 during deployment to prevent an unwanted phase transformation to martensite.

According to an alternative aspect, the shape memory material of the reinforcement member 35 may have a value of A_{f} which is less than or equal to body temperature (37° C) so that the reinforcement member 35 may transform to an austenitic structure and assume the deployment configuration (e.g., pigtail) when warmed up to about body temperature. For example, the shape memory material may have a value of A_{f} in the range of from about 27° C. to about 37° C. Alternatively, A_{f} may range from about 32° C. to about 37° C. It is also possible for A_{f} to be less than 27° C. According to this aspect, the stent 5 (reinforcement member 35) may require cooling during delivery to prevent the martensitic structure from prematurely transforming to austenite. As the stent 5 is being advanced in the body, the cooling may entail keeping the reinforcement member 35 at a temperature below Aₛ by, for example; flushing a biocompatible cold fluid (e.g., cold saline) through the delivery system. To accommodate the flow of fluid, the wire guide 55 or guiding catheter may include a lumen.

The reinforcement member 35 may be formed of a resilient material having a high yield stress and a low modulus of elasticity. A stress-strain plot for the material may include a large area under the linear (elastic) portion of the curve. Such materials may be capable of higher amounts of elastic deformation than typical metals and alloys. An example of a resilient material is a high-carbon spring steel. According to this example, the resilient material may change from one configuration to another by the application and removal of stress. For example, the stent may be held in the delivery configuration by a constraining member (*e.g*., an underlying stiff guide wire or an overlying sheath) for delivery into the duct. As noted above, due to the elasticity of the resilient material, the delivery configuration may be pliable and may vary during delivery to accommodate undulations and/or tortuosity within the vessel or duct. The stent may revert to the deployment configuration upon removal or retraction of the constraining member when in place at the treatment site.

A method of deploying a nonexpandable stent in a body passageway is set forth herein. A stent having a tubular body including a distal portion, a proximal portion, and a central longitudinal portion between the distal and proximal portions, is provided. The tubular body includes at least one securing element. The securing element includes a reinforcement member comprising a shape memory material. The stent is delivered into the vessel or duct of interest for positioning at a treatment site. The securing element has a delivery configuration of the reinforcement member for delivery of the stent to the treatment site. Preferably, the delivery configuration of the reinforcement member is pliable to accommodate undulations and/or tortuosity within the vessel or duct. According to a preferred example, the reinforcement member may include a martensitic phase of the shape memory material in the delivery configuration.

An introduction system that includes an endoscope, a wire guide, an optional guiding catheter, and a pushing catheter may be used to deliver the stent to the treatment site. According to some aspects of the method, a constraining member (*e.g.*, a sheath or wire guide) may be needed to maintain the delivery configuration of the reinforcement member as the stent is passed through the body.

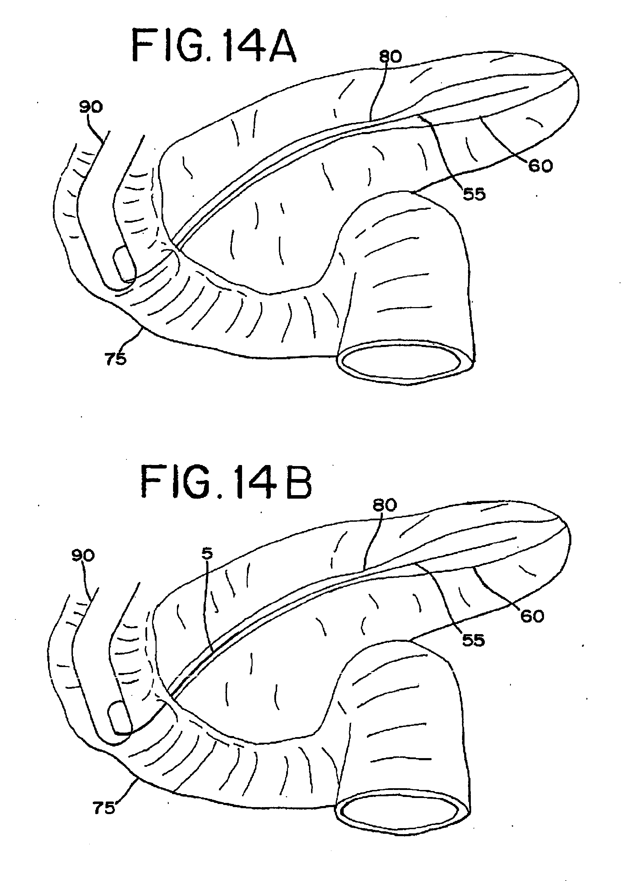

Referring to Figure 14A, the wire guide 55 may be advanced through the endoscope 90 positioned in the duodenum 75 and directed into the duct 60 of interest. A distal end of the wire guide 55 may be placed distal of the treatment site (*e.g*., stricture) 80. The stent 5 may then be advanced over the wire guide 55 through the endoscope 90 for placement in the duct 60 in the vicinity of the treatment site 80, as shown in Figure 14B. The procedure may be performed under fluoroscopic guidance using radiopaque markers attached to the stent 5 and/or guiding catheter.

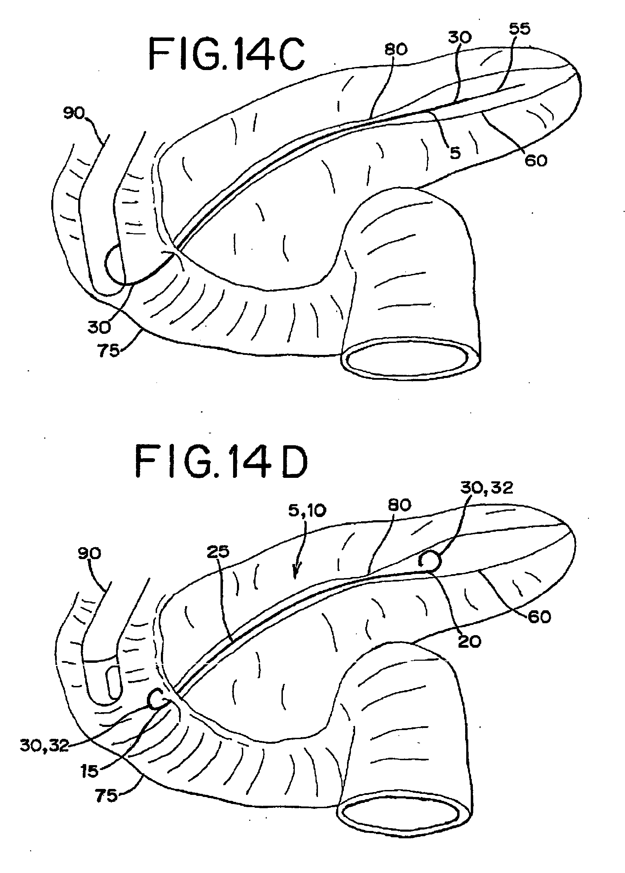

Figure 14C shows the stent 5 in position for deployment in the duct 60. The wire guide 55 and the optional guiding catheter (not visible in figures) may be removed to deploy the stent. To deploy the stent 5, the one or more reinforcement members 35 and consequently the one or more securing elements 30 attain a deployment configuration, as shown in Figure 14D. According to one aspect of the method, deployment comprises a phase change of the shape memory material of the reinforcement member 35 from martensite to austenite. The stent 5 may be deployed superelastically by removal of a constraining member 30, according to one aspect. For example, a sheath overlying the stent 5 or a stiff wire guide 55 underlying the stent 5 may be retracted to trigger deployment of the one or more securing elements 30. According to another example, the stent 5 may be deployed by a change in temperature of the shape memory material of the reinforcement member 35. The stent 5 may be warmed such that the shape memory material of the reinforcement member 35 reaches or exceeds a temperature of Aₛ or, preferably, A_{f}. According to an example in which Aₛ or A_{f} is less than or equal to body temperature, the reinforcement member 35 may be warmed to the deployment configuration by the temperature of the body vessel or duct. Alternatively, according to an example in which Aₛ or A_{f} is above body temperature, the warming of the reinforcement member 35 may occur by circulating a warming fluid through the delivery system of the stent 5. Once the stent 5 is deployed, the securing element 30 has a deployment configuration that includes, for example, a flap 34 or a pigtail 32.

Preferably, the shape memory material is an equiatomic or near-equiatomic binary nickel-titanium alloy (e.g., Nitinol). Such nickel-titanium compositions are known in the art and may be obtained from a number of commercial sources, including Special Metals Corp. (New Hartford, NY), Memry Corp. (Bethel, CT), and Johnson Matthey, Inc. (West Chester, PA). The shape memory material may further include additional alloying elements, such as ternary or quaternary additions. Such additional alloying elements may be selected from the group consisting of aluminum, boron, chromium, cobalt, copper, gold, hafnium, iron, manganese, niobium, palladium, platinum, tantalum, tungsten, vanadium, and zirconium.

A method of making the nonexpandable stent according to the present disclosure is also set forth herein. At least one reinforcement member (e.g., a wire) comprising a shape memory material, is provided. The shape memory/superelastic properties are imparted to the reinforcement member prior to the formation of the stent. a heat treatment is employed to impart a "memory" of a desired final shape and to optimize the shape memory/superelastic properties of the reinforcement member. As is known by those of ordinary skill in the art, the number, duration and the temperature of the heat treatments may alter the transformation temperatures of the shape memory material. Heat treatment temperatures of 350°C to 550°C are typically employed.

The reinforcement member is then held adjacent to a mandrel with a desired spacing therebetween along a length of the reinforcement member. A fixture may be employed to hold the reinforcement member adjacent to the mandrel at the desired spacing and in a desired configuration. It may be advantageous to cool the reinforcement member to below M_{f} of the shape memory material prior to positioning the member adjacent to the mandrel, so as to improve the ease of deforming and restraining the reinforcement member. The spacing between the mandrel and the reinforcement member may be variable or constant along the length. The spacing may lie in the range of from about 0.01 mm to about 2 mm, for example, depending on the desired wall thickness of the stent and the preferred placement of the reinforcement member within the wall of the stent. Preferably, the reinforcement member is positioned equidistant between the outer and inner walls of the formed stent. According to one embodiment, the spacing between the mandrel and the reinforcement member lies in the range of from about 0.05 mm to about 1 mm.

A coating solution is then applied to the reinforcement member and the mandrel to form the nonexpandable stent. According to one embodiment, the coating solution may be applied by dipping. Alternatively, the coating solution may be applied by spraying, spinning, or other coating methods known in the art. The coating solution may be applied at ambient temperature.

After application to the reinforcement member and the mandrel, the coating solution is cured to form a polymer layer thereon. The curing may be carried out by any curing method known in the art. For example, heating, radiation (*e.g*., ultraviolet, electron beam) or chemicals may be used to carry out the curing. The applying of the coating solution and the curing steps are repeated sequentially to form successive polymer layers on the reinforcement member and the mandrel. For example, ten to 20 successive dipping and curing steps may be used. In this way, the nonexpandable stent may be formed having a desired wall thickness. Once the desired wall thickness is obtained, the mandrel may be removed, thereby forming a lumen of the stent. The reinforcement member may be cut to a desired length.

Upon cutting the stent to length, the ends of the reinforcement member are preferably embedded within the stent or flush with the ends of the stent. If desired, a UV curable adhesive may be applied to one or both ends of the stent to form a polymeric layer over exposed portions of the reinforcement member, thereby reducing the possibility of trauma between the reinforcement member and the vessel or duct wall during delivery of the stent. It is also possible to overmold the cut stent with another polymer as a means of covering exposed portions of the reinforcement member.

A nonexpandable stent and a method of making the stent have been disclosed. The stent has at least one securing element that comprises a reinforcement member formed of a shape memory material. The stent deploys in the vicinity of a stricture in a body passageway, such as the pancreatic duct, by a change in configuration of the securing element from a delivery configuration to a deployment configuration. A phase change of the shape memory material resulting from a change in stress and/or temperature drives deployment of the stent, according to one aspect. The nonexpandable stent of the present disclosure may be formed in an inexpensive, ambient temperature coating process. In contrast, conventional pancreatic or biliary stents are generally manufactured by extrusion at elevated temperatures followed by heat forming to set the deployment configuration. Accordingly, conventional pancreatic or biliary stents are generally limited to thermoplastic polymers. In contrast, the stent of the present disclosure may be formed from a wide range of polymers that have desirable properties, such as, for example, Thoralon.

It is therefore intended that the foregoing detailed description be regarded as illustrative rather than limiting, and that it be understood that it is the following claims, that are intended to define the scope of this invention.

## Claims

1. A nonexpandable stent (5) comprising:
a tubular body (10) having a distal portion (20), a proximal portion (15), central longitudinal portion (25) between the distal and proximal portions, and a substantially nonexpandable diameter,
wherein the tubular body comprises at least one securing element (30) integrally formed with the tubular body, the tubular body comprising a polymer, the securing element including a reinforcement member (35) comprising a nickel-titanium alloy shape memory material;
wherein the securing element comprises a first configuration of the reinforcement member in which first configuration the reinforcement member comprises an martensitic phase of the nickel-titanium alloy for delivery to a treatment site within a body vessel, and a second configuration of the reinforcement member for deployment at the treatment site, in which second configuration the reinforcement member is an austenitic phase of the nickel-titanium alloy,
**characterized by** said reinforcement member being embedded in said polymer and extending along at least a portion of the length of said tubular body.

2. The stent according to claim 1. wherein the nickel-titanium alloy has an austenitic final temperature (A_{f}) which is less than or equal to body temperature.

3. The stent according to claim 1 or 2, wherein the reinforcement member is a wire.

4. The stent according to claim 1, wherein the securing element is configured to facilitate movement of the stent through the body vessel when the reinforcement member is in the first configuration, and wherein the securing element is configured to inhibit movement of the stent with respect to the body vessel when the reinforcement member is in the second configuration.

5. The stent according to claim 1, wherein the securing element is disposed in at least one of the distal portion and the proximal portion of the tubular body.

6. The stent according to claim 1, wherein the securing element extends in a direction of the central longitudinal portion when the reinforcement member is in the first configuration, and wherein the securing element extends in a direction away from the central longitudinal portion when the reinforcement member is in the second configuration.

7. The stent according to claim 1, wherein the securing element comprises a curve when the reinforcement member is in the second configuration.

8. The stent according to claim 1, wherein the securing element is a bend in the central longitudinal portion when the reinforcement member is in the second configuration.

9. The stent of claim 1 comprising two or more reinforcement members.

10. The stent according to claim 1, wherein the polymer is a polyurethane base polymer blended with a siloxane containing surface modifying additive.

11. A method of making a nonexpandable stent (5) in accordance to claim 1, the method comprising:
providing at least one reinforcement member (35) comprising a nickel-titanium alloy shape memory material
heating the reinforcement member to impart a memory of a desired final shape to the reinforcement member;
after the heating treating, holding the reinforcement member adjacent to a mandrel with a spacing therebetween along a length of the reinforcement member;
applying a coating solution to the reinforcement member and the mandrel, curing the coating solution, thereby obtaining a polymer layer on the reinforcement member and the mandrel;
removing the mandrel, thereby forming a nonexpandable stent including the reinforcement member within in a wall thereof, wherein the applying and curing are repeated sequentially to form successive polymer layers on the reinforcement member and the mandrel before removing the mandrel.

12. A method according to claim 11 wherein said spacing is variable along the length of the reinforcement member.

13. A method according to claim 11 where the heating of the reinforcement member is carried out at a temperature of between 350°C and 550°C.

## Patentansprüche

1. Nicht-expandierbarer Stent (5), umfassend:
einen röhrenförmigen Körper (10) mit einem distalen Abschnitt (20), einem proximalen Abschnitt (15) einem mittleren Längsabschnitt (25) zwischen den distalen und proximalen Abschnitten und einen im Wesentlichen nichtexpandierbaren Durchmesser,
worin der röhrenförmige Körper zumindest ein Befestigungselement (30) umfasst, das einstückig mit dem röhrenförmigen Körper geformt ist, wobei der röhrenförmige Körper ein Polymer umfasst, das Befestigungselement ein Verstärkungselement (35) aufweist, das ein Nickel-Titan-Legierungsmaterial mit Formgedächtnis umfasst;
worin das Befestigungselement eine erste Konfiguration des Verstärkungselements umfasst, in welcher ersten Konfiguration das Verstärkungselement eine martensitische Phase der Nickel-Titan-Legierung zur Abgabe an einen Behandlungssitus in einem Blutgefäß und eine zweite Konfiguration des Verstärkungselements zum Einsetzen an dem Behandlungssitus umfasst, wobei die zweite Konfiguration des Verstärkungselements eine austenitische Phase der Nickel-Titan-Legierung ist,
**dadurch gekennzeichnet, dass** das Verstärkungselement in dem Polymer eingebettet ist und sich zumindest entlang eines Teils der Länge des röhrenförmigen Körpers erstreckt.

2. Stent nach Anspruch 1, worin die Nickel-Titan-Legierung eine austenitische Endtemperatur (A_{f}) aufweist, die kleiner als die Körpertemperatur ist oder dieser gleicht.

3. Stent nach Anspruch 1 oder 2, worin das Verstärkungselement ein Draht ist.

4. Stent nach Anspruch 1, worin das Befestigungselement zur Erleichterung der Bewegung des Stents durch das Körpergefäß ausgelegt ist, wenn sich das Verstärkungselement in der ersten Konfiguration befindet, und worin das Befestigungselement zur Unterdrückung der Bewegung des Stents bezüglich des Körpergefäßes ausgelegt ist, wenn sich das Verstärkungselement in der zweiten Konfiguration befindet.

5. Stent nach Anspruch 1, worin das Befestigungselement zumindest in dem distalen Abschnitt und/oder proximalen Abschnitt des röhrenförmigen Körpers angeordnet ist.

6. Stent nach Anspruch 1, worin sich das Befestigungselement in einer Richtung des mittleren Längsabschnitts erstreckt, wenn sich das Verstärkungselement in der ersten Konfiguration befindet, und worin sich das Befestigungselement in einer Richtung von dem mittleren Längsabschnitt weg erstreckt, wenn sich das Verstärkungselement in der zweiten Konfiguration befindet.

7. Stent nach Anspruch 1, worin das Befestigungselement eine Kurve umfasst, wenn sich das Verstärkungselement in der zweiten Konfiguration befindet.

8. Stent nach Anspruch 1, worin das Befestigungselement eine Biegung in dem mittleren Längsabschnitt ist, wenn sich das Verstärkungselement in der zweiten Konfiguration befindet.

9. Stent nach Anspruch 1, der zwei oder mehr Verstärkungselemente umfasst.

10. Stent nach Anspruch 1, worin das Polymer ein Polymer auf Polyurethanbasis im Gemisch mit einem siloxanhaltigen oberflächenmodifizierenden Zusatzstoff.

11. Verfahren zur Herstellung eines nichtexpandierbaren Stents (5) nach Anspruch 1, worin das Verfahren Folgendes umfasst:
Bereitstellung zumindest eines Verstärkungselements (35) aus einem Nickel-Titan-Legierungsmaterial mit Formgedächtnis;
Erwärmen des Verstärkungselements, um ihm ein Gedächtnis für eine gewünschte Endgestalt des Verstärkungselements zu verleihen;
nach der Wärmebehandlung Halten des Verstärkungselements neben einem Mandrin mit einem Abstand dazwischen entlang einer Länge des Verstärkungselements;
Aufbringen einer Beschichtungslösung auf das Verstärkungselement und den Mandrin, Aushärten der Beschichtungslösung, wodurch eine Polymerschicht auf dem Verstärkungselement und dem Mandrin erhalten wird;
Entfernen des Mandrins, wodurch ein nicht-expandierbarer Stent geformt wird, der das Verstärkungselement in einer Wand aufweist, worin der Aufbring- und Aushärtevorgang sequentiell wiederholt wird, um aufeinanderfolgende Polymerschichten auf dem Verstärkungselement und dem Mandrin vor Entfernen des Mandrins zu formen.

12. Verfahren nach Anspruch 11, worin der Abstand über die Länge des Verstärkungselements variabel ist.

13. Verfahren nach Anspruch 11, worin die Erwärmung des Verstärkungselements bei einer Temperatur zwischen 350°C und 550°C durchgeführt wird.

## Revendications

1. Implant intravasculaire non expansible (5) comportant :
un corps tubulaire (10) doté d'une partie distale (20), d'une partie proximale (15) et d'une partie longitudinale (25) située entre la partie distale et la partie proximale, le diamètre étant essentiellement non expansible,
le corps tubulaire comportant au moins un élément de fixation (30) formé d'un seul tenant avec le corps tubulaire, le corps tubulaire comportant un polymère, l'élément de fixation comportant un élément de renfort (35) qui présente un matériau à mémoire de forme basé sur un alliage de nickel et titane,
l'élément de fixation comportant une première configuration de l'élément de renfort dans laquelle l'élément de renfort comporte une phase martensitique de l'alliage de nickel et de titane, configuration dans laquelle il est placé sur un site de traitement à l'intérieur d'un vaisseau du corps, et une deuxième configuration de l'élément de renfort dans laquelle il est déployé sur le site de traitement, l'élément de renfort présentant une phase austénitique de l'alliage de nickel et de titane dans la deuxième configuration, **caractérisé en ce que**
ledit élément de renfort est incorporé dans ledit polymère et s'étend sur au moins une partie de la longueur dudit corps tubulaire.

2. Implant intravasculaire selon la revendication 1, dans lequel l'alliage de nickel et de titane présente une température austénitique finale (A_{f}) inférieure ou égale à la température du corps.

3. Implant intravasculaire selon les revendications 1 ou 2, dans lequel l'élément de renfort est un fil.

4. Implant intravasculaire selon la revendication 1, dans lequel l'élément de fixation est configuré pour permettre le déplacement de l'implant intravasculaire dans le vaisseau du corps lorsque l'élément de renfort se trouve dans la première configuration, l'élément de fixation étant configuré pour empêcher le déplacement de l'implant intravasculaire par rapport au vaisseau du corps lorsque l'élément de renfort se trouve dans la deuxième configuration.

5. Implant intravasculaire selon la revendication 1, dans lequel l'élément de fixation est disposé dans la partie distale et/ou dans la partie proximale du corps tubulaire.

6. Implant intravasculaire selon la revendication 1, dans lequel l'élément de fixation s'étend dans une direction de la partie longitudinale centrale lorsque l'élément de renfort est dans la première configuration, l'élément de fixation s'étendant dans une direction différente de la portion longitudinale centrale lorsque l'élément de renfort est dans la deuxième configuration.

7. Implant intravasculaire selon la revendication 1, dans lequel l'élément de fixation comporte une courbe lorsque l'élément de renfort est dans la deuxième configuration.

8. Implant intravasculaire selon la revendication 1, dans lequel l'élément de fixation présente une courbe dans la partie longitudinale centrale lorsque l'élément de renfort est dans la deuxième configuration.

9. Implant intravasculaire selon la revendication 1, comportant deux ou plusieurs éléments de renfort.

10. Implant intravasculaire selon la revendication 1, dans lequel le polymère est un polymère à base de polyuréthane mélangé avec un siloxane qui contient un additif de modification de surface.

11. Procédé de fabrication d'un implant intravasculaire non expansible (5) selon la revendication 1, le procédé comportant les étapes qui consistent à :
prévoir au moins un élément de renfort (35) comportant un matériau à mémoire de forme à base d'un alliage de nickel et de titane,
chauffer l'élément de renfort pour conférer à l'élément de renfort la mémoire d'une forme finale souhaitée,
après le traitement thermique, maintenir l'élément de renfort en position adjacente à un mandrin en respectant un écartement suivant la longueur de l'élément de renfort,
appliquer une solution de revêtement sur l'élément de renfort et le mandrin, faire durcir la solution de revêtement pour ainsi obtenir une couche polymère sur l'élément de renfort et sur le mandrin,
enlever le mandrin pour ainsi former un implant intravasculaire non expansible qui contient l'élément de renfort dans une de ses parois, l'application et le durcissement étant répétés successivement de manière à former des couches polymères successives sur l'élément de renfort et sur le mandrin avant d'enlever le mandrin.

12. Procédé selon la revendication 11, dans lequel ledit écartement varie suivant la longueur de l'élément de renfort.

13. Procédé selon la revendication 11, dans lequel le chauffage de l'élément de renfort est exécuté à une température comprise entre 350°C et 550°C.
